(19)

Europäisches Patentamt

European Patent Office

Office européen des brevets

(11) **EP 4 733 753 A1**

(12) **EUROPEAN PATENT APPLICATION**

(43) Date of publication:
 **29.04.2026 Bulletin 2026/18**

(21) Application number: **24209138.7**

(22) Date of filing: **28.10.2024**

(51) International Patent Classification (IPC):
 **G01N 27/30** (2006.01)   **A61B 5/00** (2006.01)

(52) Cooperative Patent Classification (CPC):
 **G01N 27/301;** A61B 5/00

(84) Designated Contracting States:
 **AL AT BE BG CH CY CZ DE DK EE ES FI FR GB GR HR HU IE IS IT LI LT LU LV MC ME MK MT NL NO PL PT RO RS SE SI SK SM TR**
 Designated Extension States:
 **BA**
 Designated Validation States:
 **GE KH MA MD TN**

(71) Applicants:
 • **Imec VZW**
  **3001 Leuven (BE)**
 • **Stichting IMEC Nederland**
  **5656 AE Eindhoven (NL)**

(72) Inventors:
 • **Malissovas, Anastasios**
  **5642 GH Eindhoven (NL)**
 • **Henry, Olivier**
  **1950 Kraainem (BE)**

(74) Representative: **Körfer, Thomas**
 **Mitscherlich PartmbB**
 **Patent- und Rechtsanwälte**
 **Karlstraße 7**
 **80333 München (DE)**

(54) **WAFER-SCALE FABRICATION METHOD FOR A NON-CYTOTOXIC, DRIFT-COMPENSATED REFERENCE ELECTRODE IN HEALTH AND BIOTECHNOLOGY APPLICATIONS**

(57)    A method (100) for wafer-scale fabrication of a non-cytotoxic reference electrode for various types of sensors, including electrochemical sensors, biosensors, and other devices used for analyte sensing is provided. The method comprises the steps of forming (101) at least one electrode on a substrate, forming (102) one or more metal or metal oxide layers on top of the at least one electrode, functionalizing (103) the one or more metal or metal oxide layers, providing (104) a membrane on top of the one or more metal or metal oxide layers to confine ions diffused from the one or more metal or metal oxide layers, forming (105) a reservoir on top of the membrane, filling (106) the reservoir with a reference electrolyte, and providing (107) at least one sensor inside the reservoir being in contact with the reference electrolyte to measure a total ion concentration and/or a specific ion concentration and/or a temperature of the reference electrolyte, enabling correction for sensor drift.

100

101 — forming at least one electrode on a substrate

102 — forming one or more metal or metal oxide layers on top of the at least one electrode

103 — functionalizing the one or more metal or metal oxide layers

104 — providing a membrane on top of the one or more metal or metal oxide layers to confine ions diffused from the one or more metal or metal oxide layers

105 — forming a reservoir on top of the membrane

106 — filling the reservoir with a reference electrolyte

107 — providing at least one sensor inside the reservoir being in contact with the reference electrolyte to measure a total ion concentration and/or a specific ion concentration and/or a temperature of the reference electrolyte

**Fig. 1**

EP 4 733 753 A1

**Description**

The invention relates to true reference electrodes for various types of sensors, including electrochemical sensors, biosensors, and other devices used for analyte sensing, especially to fabricate a non-cytotoxic true reference electrode using microfabrication technology and a cap to create a reservoir to house its reference electrolyte.

Generally, the reference electrode is an essential component for various types of sensors, including electrochemical sensors, biosensors, and other devices used for analyte sensing. Potentiometric sensors, such as a pH sensor, generate a voltage difference between an indicating or measuring electrode and a reference electrode. The interfacial potential of the latter shall be known, stable and independent of the test fluid composition. Such an electrode system is known as a true reference electrode.

The most used reference electrode is an electrode consisting of a mixture of bare silver (Ag) and silver chloride (AgCl) immersed in a reservoir with the (reference) electrolyte solution, which has a constant chloride (Cl) concentration. A liquid junction separates the inner reservoir from the solution under test, while still providing ionic contact to the test fluid.

For example, the document US 2024/0041364 Al discloses an integrated reference electrode, a method of manufacturing an integrated reference electrode, and an ion selective membrane. The integrated reference electrode comprises a reference electrode in combination with a hygroscopic hydrogel electrolyte impregnated and contained in a porous framework, wherein the hygroscopic hydrogel electrolyte is adapted to contact the reference electrode when in a hydrated state.

However, US 2024/0041364 A1 does not address sensor drift in miniaturized reference electrodes, especially due to the inevitable leaching of the chloride and silver ions from the reference electrode. In microfabricated devices, this leaching will manifest as sensor drift, affecting the sensor performance.

Accordingly, an object of the invention is to provide a method for manufacturing a microfabricated reference electrode, a reference electrode, and an electrochemical sensor for mitigating the aforementioned sensor drift. Another object is to prevent the diffusion of silver ions into the reference electrolyte solution to prevent contamination of the liquid junction and/or test fluid, particularly in applications containing biological media such as proteins, with which silver can react.

These and other objects are solved by the features of the first independent claim for the method, by the features of the second independent claim for the reference electrode, and by the features of the third independent claim for the electrochemical sensor. The dependent claims contain further developments.

According to a first aspect of the invention, a method is provided for manufacturing a microfabricated reference electrode. The method comprises the steps of forming at least one electrode on a substrate, forming one or more metal or metal oxide layers on top of the at least one electrode, functionalizing the one or more metal or metal oxide layers, providing a membrane on top of the one or more metal or metal oxide layers to confine ions diffused from the one or more metal or metal oxide layers, forming a reservoir on top of the membrane, filling the reservoir with a reference electrolyte, and providing at least one sensor inside the reservoir being in contact with the reference electrolyte to measure a total ion concentration and/or a specific ion concentration and/or a temperature of the reference electrolyte.

Preferably, the step of functionalizing the one or more metal or metal oxide layers comprises chlorinating the one or more metal or metal oxide layers.

Therefore, the diffusion limiting membrane advantageously avoids a direct contact between the reference electrolyte and the one or more metal layers, i.e., the leaching of the chloride and silver ions, by confining ions diffused from the one or more metal or metal oxide layers, thereby preventing contamination of the liquid junction and the test fluid.

Furthermore, the sensor advantageously measures the total ion concentration and/or a specific ion concentration of the reference electrolyte, e.g., the concentration of the chloride ions, which allows compensating for the drift in the reference electrode, thereby ensuring more accurate and stable measurements.

Advantageously, by preventing metal ions, e.g., silver, and other contaminants from leaching out of the membrane, the invention prevents chemical reactions with proteins in the test fluid, (e.g., in a bioreactor or a flow cell). This effectively mitigates bioprocess contamination and complies with the required ISO 10993-part-5 standard for cytotoxicity testing.

Preferably, the step of forming the one or more metal or metal oxide layers comprises depositing the one or more metal or metal oxide layers on top of the at least one electrode, especially using electrochemical deposition, evaporation, dispensing and printing techniques.

Preferably, the metal or metal oxide layers are formed using electrochemical deposition, as this method ensures uniformity and adherence to the underlying metal layers. For example, to achieve the electrochemical deposition of the one or more metal or metal oxide layers, the electrodes may be patterned to create deposition tracts with a uniform resistance across the wafer, which may allow uniform deposition for all tracks.

Preferably, the step of providing the membrane on top of the one or more metal or metal oxide layers comprises the steps of forming a top layer on top of the substrate and/or its passivation, creating one or more trenches in the top layer, and depositing the membrane on the top layer along the one or more trenches. Advantageously, the deposition of the membrane on the top layer can be accurately and effortlessly controlled. In addition, the trenches may effectively confine the membrane on the top layer and guiding its deposition along the defined areas.

Preferably, the step of forming the reservoir on top of the membrane comprises the steps of providing a cover surrounding the substrate comprising an outer surface, an inner surface and a hollow cavity, bonding the inner surface of the cover to the top layer, thereby protecting the interconnects, filling the hollow cavity with the reference electrolyte, and providing a porous frit at the outer surface of the cover to limit the diffusion of the reference electrolyte, e.g., to control the diffusion rate of the reference electrolyte. Advantageously, the reservoir can be formed in the cover, which can be used for packaging the sensor die, enabling seamless integration into fluidic systems or probe devices.

Preferably, the step of bonding the inner surface of the cover to the top layer comprises bonding the inner surface of the cover to the top layer using room temperature bonding, thereby preserving sensitive materials in the surroundings, like enzymatic sensors. Advantageously, an accurate alignment for the bonding can be achieved with no thermal stress on the neighboring sensitive sensor materials.

Preferably, the inner surface of the cover comprises an inlet and an outlet. In this regard, the step of filling the reservoir with the reference electrolyte comprises filling the hollow cavity of the cover with a solid electrolyte, e.g., the reference electrolyte in the form of a gel solution, through the inlet or the outlet.

Alternatively, the step of filling the reservoir with the reference electrolyte comprises filling the hollow cavity of the cover with a solid electrolyte, e.g., the reference electrolyte in the form of a gel solution, through the outer surface of the cover before providing the porous frit at the outer surface.

Advantageously, the reservoir can be filled post-manufacturing with a gel solution, thereby allowing dry storage for a longer period, e.g., up to one year.

Additionally or alternatively, the solid electrolyte inside of the hollow cavity of the cover can be replaced through the inlet or outlet, thereby allowing sensor recalibration without the need to detach the reference electrode from its integrated system, thereby prolonging the lifetime of the electrode.

According to a second aspect of the invention, a reference electrode is provided. The reference electrode comprises at least one electrode on a substrate. In addition, the reference electrode comprises one or more metal or metal oxide layers on top of the at least one electrode, In this regard, the one or more metal or metal oxide layers are functionalized. Furthermore, the reference electrode comprises a membrane on top of the one or more metal or metal oxide layers. In this regard, the membrane is configured to confine ions diffused from the one or more metal or metal oxide layers.

Moreover, the reference electrode comprises a cover surrounding the substrate on top of the membrane. In this regard, the cover comprises a hollow cavity acting as a reservoir filled with a reference electrolyte, and an outer surface comprising a porous frit configured to limit the diffusion of the reference electrolyte, e.g., to control the diffusion rate of the reference electrolyte, while providing ionic contact with the test fluid.

The reference electrode further comprises at least one sensor inside the hollow cavity being in contact with the reference electrolyte. In this regard, the at least one sensor is configured to measure a total ion concentration and/or a specific ion concentration and/or a temperature of the reference electrolyte.

For example, the at least one sensor may comprise or be a conductivity sensor and/or a temperature sensor and/or a chloride ion sensor.

Preferably, the reference electrode comprises a top layer on top of the substrate and/or its passivation comprising one or more trenches, and the membrane is deposited on the top layer along the one or more trenches. In addition, an inner surface of the cover is bonded to the top layer on top of the membrane.

Preferably, the substrate is a silicon substrate. Alternatively, the substrate may be a glass substrate, which may be used as a substrate for forming structures by thin-film deposition on the substrate. Further alternatively, the substrate is a polyimide substrate or a sapphire substrate or a polydimethylsiloxane (PDMS) substrate or a poly-methyl-methacrylate (PMMA) substrate or a parylene substrate or a liquid crystal polymer (LCP) substrate.

Preferably, the at least one electrode is a gold electrode. Alternatively, the at least one electrode is a carbon electrode. Further alternatively, the at least one electrode is an iridium electrode. Further alternatively, the at least one electrode is a boron doped diamond electrode. Further alternatively, the at least one electrode is a platinum electrode. Further alternatively, the at least one electrode is a ruthenium electrode.

Preferably, the one or more metal or metal oxide layers are silver layers, silver chloride layers, iridium-oxide-based (IrOx) layers, or a redox polymer.

Preferably, the membrane comprises one or more layers of biocompatible, non-cytotoxic polymer materials, such as poly-hydroxyethyl-methacrylate (pHEMA), polyvinyl chloride (PVC), agarose.

Preferably, the cover comprises biocompatible materials, such as thermoplastics, such as polyetereterketon (PEEK), poly-methyl-methacrylate (PMMA), cyclic olefin copolymer (COC), polycarbonate (PC), polytetrafluoroethylene (PTFE).

Preferably, the at least one sensor is a two-electrode conductivity sensor. Alternatively, the at least one sensor is a four-electrode conductivity sensor. Further alternatively, the at least one sensor is a temperature sensor or a chloride ion sensor.

According to a third aspect of the invention, an electrochemical sensor is provided. The electrochemical sensor comprises at least one reference electrode according to the second aspect of the invention and at least one measuring electrode. In this regard, the at least one reference electrode and the at least one measuring electrode are arranged in a common housing.

For instance, the at least one measuring electrode may be configured to measure a chemical or biological parameter, such as pH, glucose, lactate, dissolved oxygen, electrolytes, oxidation-reduction potential (ORP).

It is to be noted that the method according to the first aspect corresponds to the reference electrode according to the second aspect and its implementation forms. Accordingly, the method of the first aspect may have corresponding implementation forms. Further, the electrochemical sensor according to the third aspect corresponds to the reference electrode according to the second aspect and its implementation forms. Accordingly, the electrochemical sensor of the third aspect may have corresponding implementation forms.

Exemplary embodiments of the invention are now further explained with respect to the drawings by way of example only, and not for limitation. In the drawings:

Fig. 1    shows an exemplary embodiment of the method according to the first aspect of the invention;

Fig. 2    shows an exemplary embodiment of the reference electrode according to the second aspect of the invention;

Fig. 3A    shows an exemplary wafer top view for the wafer-scale fabrication process;

Fig. 3B    shows an exemplary top view of the reference electrode;

Fig. 4    shows an exemplary embodiment of the cover; and

Fig. 5    shows an exemplary embodiment of the electrochemical sensor according to the third aspect of the invention.

Reference will now be made in detail to the embodiments of the present invention, examples of which are illustrated in the accompanying drawings. However, the following embodiments of the present invention may be variously modified and the range of the present invention is not limited by the following embodiments.

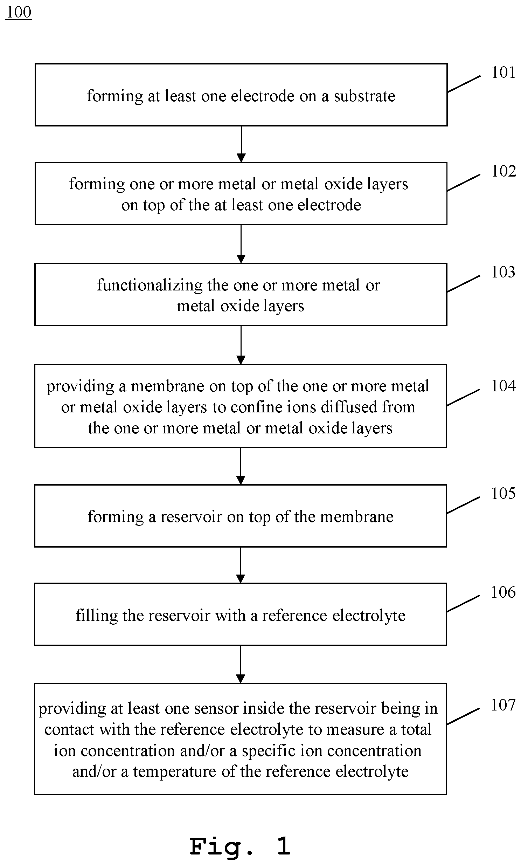

In Fig. 1, an exemplary embodiment of the method 100 according to the first aspect of the invention is illustrated. In a first step 101, an electrode is formed on a substrate. In a second step 102, one or more metal layers are formed on top of the electrode. In a third step 103, the one or more metal layers are functionalized, e.g., chlorinated. In a fourth step 104, at least one membrane is provided on top of the one or more metal layers to confine ions diffused from the one or more metal layers.

In a fifth step 105, a reservoir is formed on top of the membrane. In a sixth step 106, the reservoir is filled with a reference electrolyte. In a seventh step 107, a sensor is provided inside the reservoir being in contact with the reference electrolyte to measure a total ion concentration and/or a specific ion concentration of the reference electrolyte.

According to the method 100, the step of forming the one or more metal layers may comprise the step of depositing, e.g., electrochemically, the one or more metal layers on top of the electrode.

According to the method 100, the step of providing the membrane on top of the one or more metal layers may comprise the steps of forming a top layer on top of the substrate and/or its passivation, forming one more trenches on the top layer, and depositing the membrane on the top layer along the one or more trenches.

According to the method 100, the step of forming the reservoir on top of the membrane may comprise the steps of providing a cover surrounding the substrate comprising an outer surface, an inner surface and a hollow cavity, bonding the inner surface of the cover to the top layer, filling the hollow cavity with the reference electrolyte, and providing a porous frit at the outer surface of the cover to limit the diffusion of the reference electrolyte, e.g., to control the diffusion rate of the reference electrolyte, while ensuring ionic contact with the test fluid.

According to the method 100, the step of bonding the inner surface of the cover to the top layer may comprise the step of bonding the inner surface of the cover to the top layer using room temperature bonding.

According to the method 100, the step of filling the reservoir with the reference electrolyte may comprise the step of filling the hollow cavity of the cover with a solid electrolyte through an inlet or an outlet provided in the inner surface of the cover.

Alternatively, according to the method 100, the step of filling the reservoir with the reference electrolyte may comprise the step of filling the hollow cavity of the cover with a solid electrolyte through the outer surface of the cover before providing the porous frit at the outer surface.

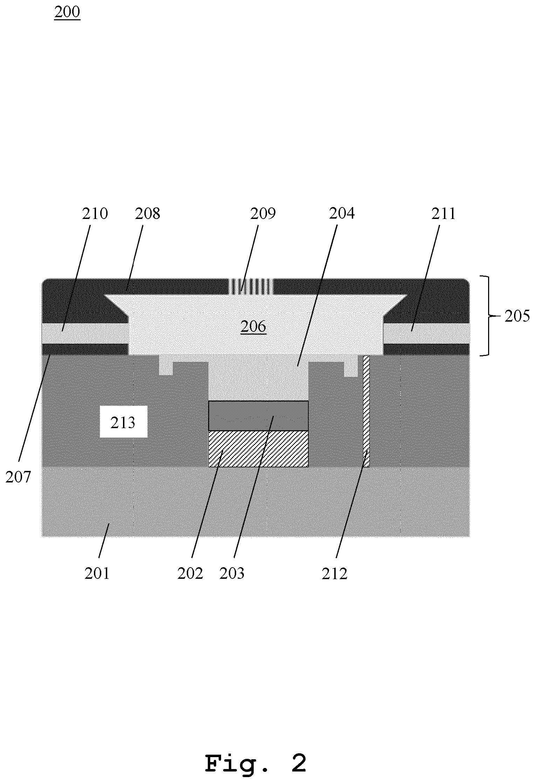

In Fig. 2, an exemplary embodiment of the reference electrode (RE) 200 according to the second aspect of the invention is illustrated.

The RE 200 may comprise a substrate 201, e.g., a silicon (Si) substrate 201, an electrode 202, e.g., a platinum (Pt) electrode 202 on the Si substrate 201, a silver (Ag) layer 203 on top of the Pt electrode 202, where the Ag layer 203 may be chlorinated. In addition, the RE 200 may comprise a membrane 204 on top of the Ag layer 203, which may confine the silver ions (Ag+) diffused from the Ag layer 203 so that a direct contact between the reference electrolyte and the Ag layer 203 is avoided.

In this regard, a top layer 213 may be provided on top of the Si substrate 201 and/or its passivation, which may comprise trenches (not shown), and the membrane 204 may be deposited on the top layer 213 along said trenches in order to house or confine the membrane 204 on top of the Ag layer 203. For example, the membrane 204 may comprise one or more layers of hydrophilic polymer materials, such as PHEMA, agarose.

Furthermore, the RE 200 may comprise a cover 205 surrounding the Si substrate 201, i.e., may be used for post-fabrication packaging of the sensor die, on top of the membrane 204. In this regard, the cover 205 may comprise a hollow cavity 206 acting as a reservoir, which may be filled with a reference electrolyte, an inner surface 207 being bonded or glued to the top layer 213 on top of the membrane 204, and an outer surface 208, which may comprise a porous frit 209 in order to diffuse the reference electrolyte.

For example, the cover 205, especially the inner surface of the cover 205, may comprise an inlet 210 and an outlet 211. In this regard, the hollow cavity 206 of the cover 205 may be filled with the reference electrolyte through the inlet 210 or the outlet 211.

Moreover, the RE 200 may comprise a sensor 212, e.g., a conductivity sensor 212, especially arranged at or near the hollow cavity 206, preferably inside of the hollow cavity 206, in order to be in contact with the reference electrolyte within the hollow cavity 206. In this regard, the conductivity sensor 212 may measure a total ion concentration and/or a specific ion concentration, especially the concentration of chloride ions, of the reference electrolyte.

For example, the conductivity sensor 212 may be a two-electrode or two-pole conductivity sensor. In this regard, the conductivity sensor 212 may comprise two plates of metal with an insulating material between them. For example, an AC voltage can be applied to the plates to achieve the current flow through the reference electrolyte. Since the voltage and current are known values, the resistance of the reference electrolyte can be calculated.

Alternatively, the conductivity sensor 212 may be a four-electrode or four-pole conductivity sensor. In this regard, the conductivity sensor 212 may comprise, in addition to the two plates of metal of the two-pole conductivity sensor, a second pair of electrodes, which may produce a known current that allows for measurement of the voltage drop across the electrodes.

Generally, the reference potential is given by the Nernst equation as:

$$E_{ref}(t) = E^{0'} + 59mV \ \log c_{res} \qquad (1)$$

Where, $E^{0'}$ is the formal potential and $c_{res}$ is the chloride concentration in the reservoir.

According to the equation (1), the reference potential remains stable if the reservoir concentration does not change over time. The reference electrode however requires an ionic connection to the test fluid, which may be formed by the liquid junction. This means that chloride ions can leach out of the reservoir. When this concentration slowly changes, the reference potential drifts over time. In practice, all reference electrodes drift, but are designed in such a way that drift is negligible.

Despite ongoing efforts to miniaturize reference electrodes using various microfabrication techniques, miniaturizing the reference electrode reservoir while achieving drift rates comparable to state-of-the-art electrodes (i.e., <0.1 mV/day) remains challenging.

In addition to addressing challenges related to miniaturization and drift, the diffusion of the reference electrolyte into the test fluid raises further considerations in biomanufacturing applications. This can lead to two major issues:

a) liquid junction contamination: the liquid junction can be contaminated with silver sulfide precipitation which leads to increased electrical resistance, and measurement errors, and
b) release cytotoxic substances: Ag+ ions react with proteins which leads to bioprocess contamination.

The diffusion limiting membrane 204 may advantageously avoid a direct contact between the reference electrolyte and the Ag layer 203, thereby preventing liquid junction contamination. Furthermore, the conductivity sensor 212 may advantageously measure the total and/or specific ion concentrations of the reference electrolyte, e.g., the concentration of the chloride ions, which allows compensating for the drift in the RE 200, thereby ensuring more accurate and stable measurements.

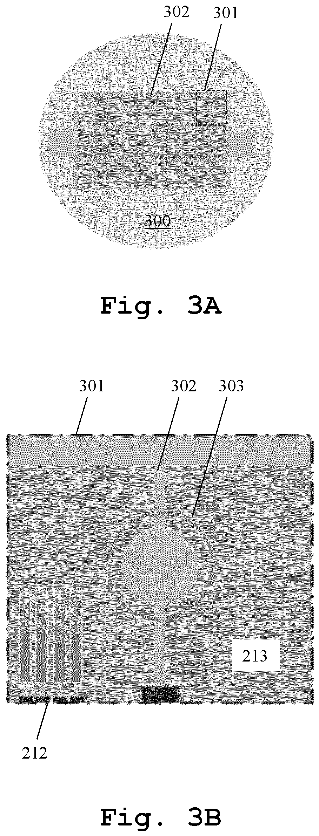

In Fig. 3A, an exemplary wafer top view for the manufacturing process is illustrated. The wafer 300 may be a silicon wafer, which may comprise a plurality of sensor dies 301. Each of the plurality of sensor dies 301 may correspond to the RE 200 without having the cover 205 on top of the sensor dies 301.

For example, the top layer 213 may be deposited or grown on the wafer 300. Afterwards, deposition tracks 302 may be created by etching with optional passivation of the top layer 213, especially with a uniform spacing across the wafer 300, thereby allowing uniform deposition for all tracks. For each of the plurality of sensor dies 301, the Pt electrode 202 may be formed, e.g., via deposition, on the wafer substrate and the subsequent Ag layer 203 on top of the Pt electrode 202, e.g., via electrochemical deposition, along the deposition tracks 302.

As such, the invention addresses the need for cost-effective batch manufacturing compatible with wafer-level process. This may also enable the integration of such components, i.e., the RE, into miniaturized devices like flow cells or microprobes.

In Fig. 3B, an exemplary top view of the RE, especially of the sensor die 301, is illustrated. For example, the trenches 303 may be formed on the top layer 213, e.g., via etching and the membrane 204 may be formed, e.g., via deposition, on the top layer 213 along the trenches 303, which may allow an effective control for the membrane deposition.

In addition, the conductivity sensor 212 may be formed on the top layer 213 such that the conductivity sensor 212 may be in contact with the content of the hollow cavity 206 of the cover 205 when the cover 205 is placed on top of the sensor die 301, especially for packaging the sensor die 301. In this example, the conductivity sensor 212 is illustrated as a four-electrode conductivity sensor. However, the conductivity sensor 212 can be a two-electrode conductivity sensor.

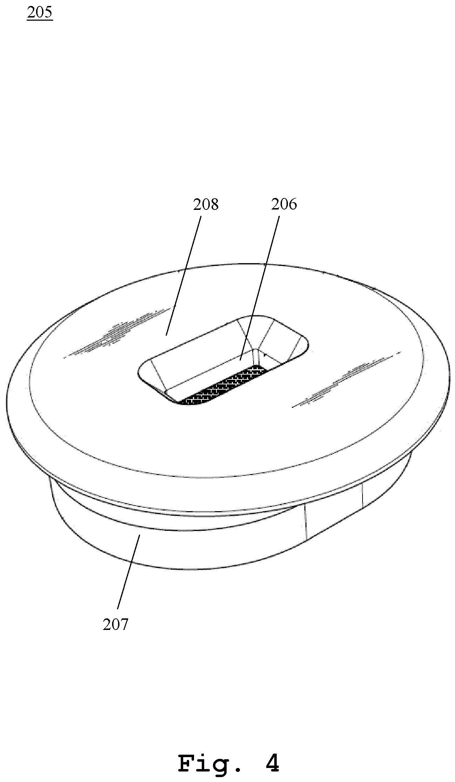

In Fig. 4, an exemplary embodiment of the cover 205 is illustrated. As discussed above along Fig. 2, the cover 205 may be arranged on top of the membrane 204 and may surround the Si substrate 201, i.e., may be used for post-fabrication packaging of the sensor die 301.

In this regard, the cover 205 may comprise the hollow cavity 206 acting as the reservoir for the reference electrolyte at a defined position with respect to the underlying sensor die 301, preferably at a central position with respect to the underlying sensor die 301. It is to be noted that the outer surface 208 is shown here without the porous frit 209, as discussed before. For example, the cover 205 may comprise biocompatible materials, such as thermoplastics, such as PEEK, PMMA, COC, PC, PTFE.

For example, the cover 205 may have the dimension of 1 mm to 50 mm, such as 1 mm to 10 mm, or such as 10 mm to 20 mm, or such as 20 mm to 30 mm, or such as 30 mm to 40 mm, or such as 40 mm to 50 mm.

For example, the cover 205 may have several different shapes, such as being a square or a circle. The shape of the cover 205 may be defined by the dimensions of the Si substrate 201 such that the entire Si substrate 201 may be covered by the cover 205.

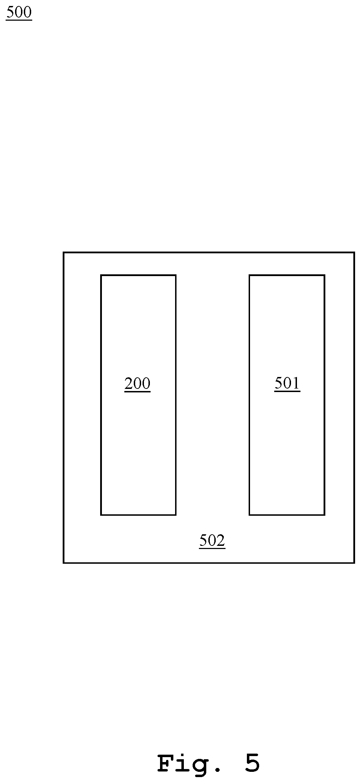

In Fig. 5, an exemplary embodiment of the electrochemical sensor 500 according to the third aspect of the invention is illustrated. The electrochemical sensor 500 may comprise the RE 200 and a measuring or indicating electrode (IE) 501. In this regard, the RE 200 and the IE 501 may be arranged in a common housing 502, e.g., a chip. For instance, the IE 501 may measure a chemical or biological parameter, such as pH, electrolytes, glucose, lactate, dissolved oxygen, oxidation-reduction potential.

It is to be noted that the electrochemical sensor 500 may comprise more than one RE 200 and/or more than one IE 501 in the housing 502. For example, the electrochemical sensor 500 may comprise at least five IEs 501, such as at least 10 IEs 501 or at least 20 IEs 501.

For example, the electrochemical sensor 500 may comprise hundreds of REs 200 and IEs 501 in any combination. In this regard, several of the REs 200 and/or the IEs 501 may measure the same parameter. Alternatively, each of the REs 200 and/or the IEs 501 may be configured to each measure a single parameter.

It is important to note that, in the description as well as in the claims, the word "comprising" does not exclude other elements or steps and the indefinite article "a" or "an" does not exclude a plurality. A single element or other unit may fulfill the functions of several entities or items recited in the claims.

It should be understood that the term "and/or" used in the specification and the appended claims of this application refers to any combination and all possible combinations of one or more associated listed items, and includes these combinations. Further, the terms first, second, etc., used herein are not to be considered limiting, but rather merely descriptive.

Although the invention has been illustrated and described with respect to one or more implementations, equivalent alterations and modifications will occur to others skilled in the art upon the reading and understanding of this specification and the annexed drawings.

In addition, while a particular feature of the invention may have been disclosed with respect to only one of several implementations, such feature may be combined with one or more other features of the other implementations as may be desired and advantageous for any given or particular application.

**Claims**

1. A method (100) for manufacturing a reference electrode, the method comprises:

   forming (101) at least one electrode on a substrate,
   forming (102) one or more metal or metal oxide layers on top of the electrode,
   functionalizing (103) the one or more metal or metal oxide layers,
   providing (104) a membrane on top of the one or more metal or metal oxide layers to confine ions diffused from the one or more metal or metal oxide layers,
   forming (105) a reservoir on top of the membrane,
   filling (106) the reservoir with a reference electrolyte, and
   providing (107) at least one sensor inside the reservoir being in contact with the reference electrolyte to measure a total ion concentration and/or a specific ion concentration and/or a temperature of the reference electrolyte.

2. The method according to claim 1,
   wherein forming one or more metal or metal oxide layers comprises depositing the one or more metal or metal oxide layers on top of the at least one electrode.

3. The method according to claim 1 or 2,
   wherein providing the membrane on top of the one or more metal or metal oxide layers comprises:

   forming a top layer on top of the substrate and/or its passivation,
   forming one more trenches in the top layer, and
   depositing the membrane on the top layer along the one or more trenches.

4. The method according to claim 3,
   wherein forming the reservoir on top of the membrane comprises:

   providing a cover surrounding the substrate comprising an outer surface, an inner surface and a hollow cavity,
   bonding the inner surface of the cover to the top layer,
   filling the hollow cavity with the reference electrolyte, and
   providing a porous frit at the outer surface of the cover to limit the diffusion of the reference electrolyte.

5. The method according to claim 4,
   wherein bonding the inner surface of the cover to the top layer comprises bonding the inner surface of the cover to the top layer using room temperature bonding.

6. The method according to claim 4,
   wherein the inner surface of the cover comprises an inlet and an outlet, and wherein filling the reservoir with the reference electrolyte comprises:

   filling the hollow cavity of the cover with a solid electrolyte through the inlet or the outlet, or
   filling the hollow cavity of the cover with a solid electrolyte through the outer surface of the cover before providing the porous frit at the outer surface.

7. A reference electrode (200) for electrochemical sensors, the reference electrode comprises:

   at least one electrode (202) on a substrate (201),
   one or more metal or metal oxide layers (203) on top of the electrode (202), wherein the one or more metal or metal oxide layers (203) are functionalized,
   a membrane (204) on top of the one or more metal or metal oxide layers (203), the membrane (204) is configured to confine ions diffused from the one or more metal or metal oxide layers (203),
   a cover (205) surrounding the substrate (201) on top of the membrane (204), wherein the cover (205) comprises a hollow cavity (206) acting as a reservoir filled with a reference electrolyte, and an outer surface (208) comprising a porous frit (209) configured to limit the diffusion of the reference electrolyte, and
   at least one sensor (212) inside the hollow cavity (206) being in contact with the reference electrolyte, the at least one sensor (212) is configured to measure a total ion concentration and/or a specific ion concentration and/or a temperature of the reference electrolyte.

8. The reference electrode according to claim 7,

   wherein the reference electrode comprises a top layer (213) on top of the substrate (201) and/or its passivation comprising one or more trenches (303), and the membrane (204) is deposited on the top layer (213) along the one or more trenches (303), and
   wherein an inner surface (207) of the cover (205) is bonded to the top layer (213) on top of the membrane (204).

9. The reference electrode according to claim 7 or 8, wherein the substrate (201) is a silicon substrate or a polyimide substrate or a glass substrate or a sapphire substrate or a polydimethylsiloxane, PDMS, substrate or a poly-methyl-methacrylate, PMMA, substrate or a parylene substrate or a liquid crystal polymer, LCP, substrate.

10. The reference electrode according to any of claims 7 to 9,
    wherein the at least one electrode (202) is a gold, carbon, iridium, boron doped diamond, platinum, or a ruthenium electrode.

11. The reference electrode according to any of claims 7 to 10,
    wherein the one or more metal or metal oxide layers (203) are silver layers, silver chloride layers, iridium-oxide-based layers, or a redox polymer.

12. The reference electrode according to any of claims 7 to 11,
    wherein the membrane (204) comprises one or more layers of polymer materials, such as poly-hydroxyethyl-methacrylate, pHEMA, polyvinyl chloride, PVC, agarose.

13. The reference electrode according to any of claims 7 to 12,
    wherein the cover (205) comprises biocompatible materials, such as thermoplastics, such as polyetereterketon, PEEK, poly-methyl-methacrylate, PMMA, cyclic olefin copolymer, COC, polycarbonate, PC, polytetrafluoroethylene, PTFE.

14. The reference electrode according to any of claims 7 to 13,

    wherein the at least one sensor (212) is a two-electrode conductivity sensor, or
    wherein the at least one sensor (212) is a four-electrode conductivity sensor, or
    wherein the at least one sensor (212) is a temperature sensor, or
    wherein the at least one sensor (212) is a chloride ion sensor.

15. An electrochemical sensor (500) comprising:

    at least one reference electrode (200) according to any of claims 7 to 14, and
    at least one measuring electrode (501),

    wherein the at least one reference electrode (200) and the at least one measuring electrode (501) are arranged in a common housing (502).

<u>100</u>

```
┌────────────────────────────────────────────────┐
│   forming at least one electrode on a substrate  │──── 101
└────────────────────────────────────────────────┘
                        │
                        ▼
┌────────────────────────────────────────────────┐
│    forming one or more metal or metal oxide layers│──── 102
│       on top of the at least one electrode        │
└────────────────────────────────────────────────┘
                        │
                        ▼
┌────────────────────────────────────────────────┐
│         functionalizing the one or more metal or │──── 103
│              metal oxide layers                   │
└────────────────────────────────────────────────┘
                        │
                        ▼
┌────────────────────────────────────────────────┐
│   providing a membrane on top of the one or more metal│──── 104
│   or metal oxide layers to confine ions diffused from │
│        the one or more metal or metal oxide layers    │
└────────────────────────────────────────────────┘
                        │
                        ▼
┌────────────────────────────────────────────────┐
│        forming a reservoir on top of the membrane │──── 105
└────────────────────────────────────────────────┘
                        │
                        ▼
┌────────────────────────────────────────────────┐
│       filling the reservoir with a reference electrolyte│──── 106
└────────────────────────────────────────────────┘
                        │
                        ▼
┌────────────────────────────────────────────────┐
│   providing at least one sensor inside the reservoir being in│──── 107
│   contact with the reference electrolyte to measure a total  │
│    ion concentration and/or a specific ion concentration     │
│    and/or a temperature of the reference electrolyte         │
└────────────────────────────────────────────────┘
```

Fig. 1

Fig. 2

Fig. 3A

Fig. 3B

205

208    206

207

Fig. 4

500

200    501

502

Fig. 5

Europäisches
Patentamt

European
Patent Office

Office européen
des brevets

**EUROPEAN SEARCH REPORT**

**Application Number**

EP 24 20 9138

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2011/056831 A1 (KENDIG MARTIN W [US] ET AL) 10 March 2011 (2011-03-10) <br> * abstract * <br> * figure 1 * <br> * paragraphs [0017] - [0021] * <br> ----- | 1-15 | INV. <br> G01N27/30 <br><br> ADD. <br> A61B5/00 |
| A | WO 2016/069935 A1 (PHASE2 MICROTECHNOLOGIES LLC [US]) 6 May 2016 (2016-05-06) <br> * claims 8-20 * <br> * abstract * <br> * figures 1-4 * <br> ----- | 1-15 | |
| A | US 6 554 982 B1 (SHIN JAE HO [KR] ET AL) 29 April 2003 (2003-04-29) <br> * abstract * <br> * figure 1 * <br> * column 5, lines 15-41 * <br> ----- | 1-15 | |
| A | US 8 728 289 B2 (DINSMOOR DAVID A [US]; MATTES MICHAEL F [US] ET AL.) 20 May 2014 (2014-05-20) <br> * abstract * <br> * figure 2 * <br> * column 4, line 65 - column 5, line 15 * <br> ----- <br> -/-- | 1-15 | **TECHNICAL FIELDS SEARCHED (IPC)** <br><br> G01N <br> A61B |

The present search report has been drawn up for all claims

| Place of search | Date of completion of the search | Examiner |
|---|---|---|
| Munich | 19 March 2025 | Ruchaud, Nicolas |

EPO FORM 1503 03.82 (P04C01)

page 1 of 2

Europäisches Patentamt

European Patent Office

Office européen des brevets

# EUROPEAN SEARCH REPORT

**Application Number**

EP 24 20 9138

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SUZUKI H ET AL: "MICROFABRICATED LIQUID JUNCTION AG/AGCL REFERENCE ELECTRODE AND ITSAPPLICATION TO A ONE-CHIP POTENTIOMETRIC SENSOR", ANALYTICAL CHEMISTRY, AMERICAN CHEMICAL SOCIETY, vol. 71, no. 22, 15 November 1999 (1999-11-15), pages 5069-5075, XP000926900, DOI: 10.1021/AC990437T * abstract * * figure 1 * * pages 5070-5071, paragraph "Structure and Fabrication of the Liquid-Junction Ag/AgCl Reference Electrode" * | 1-15 | |
| A | WO 2023/180349 A1 (ANALOG DEVICES INTERNATIONAL UNLIMITED CO [IE]) 28 September 2023 (2023-09-28) * abstract * * figures 1,4A-4D * * paragraphs [0066], [0076], [0077] * | 1-15 | |
| A | EP 2 363 705 B1 (STICHTING IMEC NEDERLAND [NL]) 8 January 2020 (2020-01-08) * abstract * * paragraphs [0055], [0056] * | 1-15 | TECHNICAL FIELDS SEARCHED (IPC) |
| A | US 2018/003667 A1 (DAM VAN ANH [NL] ET AL) 4 January 2018 (2018-01-04) * abstract * * figure 5 * * paragraphs [0061], [0062] * | 1-15 | |

The present search report has been drawn up for all claims

| Place of search | Date of completion of the search | Examiner |
|---|---|---|
| Munich | 19 March 2025 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 9138

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2025

| Patent document cited in search report | | Publication date | Patent family member(s) | | Publication date |
|---|---|---|---|---|---|
| US 2011056831 | A1 | 10-03-2011 | NONE | | |
| WO 2016069935 | A1 | 06-05-2016 | CN | 107112495 A | 29-08-2017 |
| | | | EP | 3213360 A1 | 06-09-2017 |
| | | | JP | 2017532571 A | 02-11-2017 |
| | | | US | 2017261461 A1 | 14-09-2017 |
| | | | WO | 2016069935 A1 | 06-05-2016 |
| US 6554982 | B1 | 29-04-2003 | CN | 1357105 A | 03-07-2002 |
| | | | DE | 60022177 T2 | 08-06-2006 |
| | | | EP | 1171765 A1 | 16-01-2002 |
| | | | JP | 3667639 B2 | 06-07-2005 |
| | | | JP | 2002540424 A | 26-11-2002 |
| | | | KR | 20000061275 A | 16-10-2000 |
| | | | US | 6554982 B1 | 29-04-2003 |
| | | | WO | 0058720 A1 | 05-10-2000 |
| US 8728289 | B2 | 20-05-2014 | EP | 1960765 A1 | 27-08-2008 |
| | | | US | 2007138027 A1 | 21-06-2007 |
| | | | WO | 2007070226 A1 | 21-06-2007 |
| WO 2023180349 | A1 | 28-09-2023 | CN | 119137471 A | 13-12-2024 |
| | | | EP | 4496999 A1 | 29-01-2025 |
| | | | US | 2023304958 A1 | 28-09-2023 |
| | | | WO | 2023180349 A1 | 28-09-2023 |
| EP 2363705 | B1 | 08-01-2020 | EP | 2363705 A1 | 07-09-2011 |
| | | | US | 2011192720 A1 | 11-08-2011 |
| US 2018003667 | A1 | 04-01-2018 | EP | 3264074 A1 | 03-01-2018 |
| | | | US | 2018003667 A1 | 04-01-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20240041364 A1